# EUROPEAN PATENT SPECIFICATION

(11) **EP 2 451 493 B1**
(45) Date of publication and mention of the grant of the patent: **11.05.2016**
(21) Application number: 10747276.3
(22) Date of filing: 12.07.2010
(51) Int. Cl.: A61L 27/44

(54) **COMPOSITE BONE CEMENT WITH A PMMA MATRIX, CONTAINING BIOACTIVE ANTIBACTERIAL GLASSES OR GLASSCERAMICS**
VERBUNDKNOCHENZEMENT MIT PMMA-MATRIK MIT BIOAKTIVEN ANTIBAKTERIELLEN GLAS- ODER GLASKERAMIKSTOFFEN
CIMENTS OSSEUX COMPOSITES À MATRICE PMMA, CONTENANT DES VERRES OU DES VITROCÉRAMIQUES ANTIBACTÉRIENS BIOACTIFS

(30) Priority: 10.07.2009 IT TO20090518
(43) Date of publication of application: 16.05.2012
(73) Proprietor: Politecnico di Torino, 10129 Torino (IT); Università Degli Studi Di Torino, 10124 Torino (IT)
(72) Inventor: VERNE', Enrica, I-10138 TORINO (IT); MIOLA, Marta, I-10040 LA CASSA (TORINO) (IT); FERRARIS, Sara, I-10095 GRUGLIASCO (TORINO) (IT); MASSE', Alessandro, I-10122 TORINO (IT); BISTOLFI, Alessandro, I-10126 TORINO (IT); CROVA, Maurizio, I-10020 PECETTO TORINESE (TORINO) (IT); MAINA, Giovanni, I-10020 BALDISSERO TORINESE (TORINO) (IT)
(74) Representative: Rambelli, Paolo
(86) International application number: PCT/IB2010/053181
(87) International publication number: WO 2011/004355

(56) References cited:
- WO-A1-82/01990
- WO-A1-2007/144662
- BOYD ET AL: "Zinc-based glass polyalkenoate cements with improved setting times and mechanical properties", ACTA BIOMATERIALIA, ELSEVIER, AMSTERDAM, NL, vol. 4, no. 2, 9 February 2008 (2008-02-09), pages 425-431, XP022475148, ISSN: 1742-7061
- GONZALEZ CORCHON M A ET AL: "Injectable and self-curing composites of acrylic/bioactive glass and drug systems. A histomorphometric analysis of the behaviour in rabbits", BIOMATERIALS, ELSEVIER SCIENCE PUBLISHERS BV., BARKING, GB, vol. 27, no. 9, 1 March 2006 (2006-03-01), pages 1778-1787, XP025097623, ISSN: 0142-9612 [retrieved on 2006-03-01]
- MENDEZ J A ET AL: "Injectable self-curing bioactive acrylic-glass composites charged with specific anti-inflammatory/analgesic agent", BIOMATERIALS, ELSEVIER SCIENCE PUBLISHERS BV., BARKING, GB, vol. 25, no. 12, 1 May 2004 (2004-05-01), pages 2381-2392, XP004485157, ISSN: 0142-9612
- JOURNAL OF MATERIALS SCIENCE: MATERIALS IN MEDICINE MARCH 2009 KLUWER ACADEMIC PUBLISHERS NL, vol. 20, no. 3, March 2009 (2009-03), pages 733-740, XP002571364,
- JOURNAL OF MATERIALS SCIENCE: MATERIALS IN MEDICINE MARCH 2009 KLUWER ACADEMIC PUBLISHERS NL, vol. 20, no. 3, March 2009 (2009-03), pages 741-749, XP002571365,
- DER ORTHOPÄDE AUG 2004, vol. 33, no. 8, August 2004 (2004-08), pages 885-892, XP002571363, ISSN: 0085-4530
- SHINZATO S ET AL: "BIOACTIVE POLYMETHYL METHACRYLATE-BASED BONE CEMENT: COMPARISON OF GLASS BEADS, APATITE-AND WOLLASTONITE-CONTAINING GLASS-CERAMIC, AND HYDROXYAPATITE FILLERS ON MECHANICAL AND BIOLOGICAL PROPERTIES", JOURNAL OF BIOMEDICAL MATERIALS RESEARCH, WILEY, NEW YORK, NY, US, vol. 51, 1 January 2000 (2000-01-01), pages 258-272, XP008076019, ISSN: 0021-9304
- JOURNAL OF BIOMEDICAL MATERIALS RESEARCH 2002 JOHN WILEY AND SONS INC. US, vol. 59, no. 2, 2002, pages 225-232, XP002571366,

## Description

The present invention relates to acrylic polymer-based bone cements capable of promoting osteointegration and simultaneously preventing the onset of post-operative infections.

In the clinical practice, cemented fixation of orthopaedic prostheses (hip, knee) implies the application of acrylic cements, typically comprising *in situ-*polymerised polymethyl-methacrylates (PMMA).

PMMA cements are typically prepared from two components: a liquid and a powder. The liquid includes methylmethacrylate (MMA) monomers, an accelerator and/or inhibitor. The powder includes PMMA microspheres, a polymerisation initiator and/or radio-opacifying agent. These cements promote both the short-term and long-term prosthesis stability, through a mechanical anchorage, yet they do not become completely integrated with the bone tissue and exhibit poor mechanical properties. The PMMA-based cement is also used for making temporary prosthetic devices in case of revision.

A further use for this material is in spinal surgery in case of vertebral fractures, wherein PMMA is injected into the fractured vertebra for the consolidation thereof.

A significant problem in implant surgery is the possible development of infections. Indeed, the temporary prosthetic devices are often used during the infection treatment step by systemic or localised antibiotic administration.

In recent years, several expedients have been established in order to prevent the onset of periprosthetic infections; however, even though the infection rate has decreased, the problem is not completely solved. The possibility of introducing antibiotics directly into the bone cement used for fixing the prosthesis, so as to prevent the settlement of germs at the bone tissue-cement-prosthesis interface, has been known since the '70s. Since then, many investigations have been carried out to prove the effectiveness of such a method, by using different antibiotics and different bone cements.

Moreover, which way is the best way for introducing antibiotics into the cement is presently under discussion; in fact, the drug powder may be added manually during surgery to the polymer powder, or the blend may be accomplished directly during manufacture with industrial techniques.

In particular, the use of cements commercially-added with antibiotics developed mainly in European countries; in contrast, in the United States it is preferred to introduce the required antibiotic manually during surgery.

In spite of the great numbers of data present in literature, very few studies actually compare commercially produced antibiotic-added cements and cements added *in situ* with antibiotics. Even nowadays there is not a sufficient amount of extensive data that prove the effectiveness of cements added with antibiotics.

Also bio-active bone cements containing PMMA, a bio-active glass prepared from SiO₂-CaO-P₂O₅ and antibiotics, are known. Bio-active glasses described by L. L. Hench in Bio-ceramics, J. Am. Ceram. Soc. 81 [7] (1998), 1705-1728 are characterised by the fact that they are able to induce an actual chemical bond with bone tissue, thanks to their ability of interacting with biological fluids, thereby forming a hydroxyapatite layer on the surface thereof.

Even though on one hand the use thereof can improve the osteointegration characteristics, the problem remains of conferring adequate and long-lasting antibacterial properties to the bone cement.

In these cements, the antibiotic active principle normally is mixed with the polymer phase and constitutes a third phase in the cement composition.

Furthermore, the use of PMMA bone cements containing antibacterial metal salts has been proposed; WO82/01990 describes a bone cement for cementing prostheses, which contains polymethylmethacrylate, a load of glass fibre or quartz particles and a material designed to release silver ions in the form of a colloidal silver salt. In this case, too, we are dealing with a three-phase composition, in which it is difficult to regulate a sustained release of the antibacterial metal ions. Moreover, the glass phase is not bioactive.

WO 2007/144662 - Graham HR et al - Bioactive glass concerns with bioactive glass comprising Sr, which may further comprise silver oxide. Sr is introduced into glass composition in order to enhance its bioactivity. The bioactive glass is produced using melt quench or sol-gel techniques. Acta Biomaterialia - D. Boyd et al. - 2008 vol 4 pp 425-431 regards the Sr incorporation on Zn-containing glass polyalkenoate cements and the evaluation of Sr addition on mechanical properties, working and setting time. Moreover, the addition of trisodium citrate and its effect on the same mentioned properties is estimated.

Biomaterials - Gonzalez Corchon et al. - 2006 vol 27 pp 1778-1787 describes a PMMA-based cement comprising 3 different phases (PMMA, bioactive glass and drug). The anti-inflammatory properties are given by a drug. The addition of the drug fosfosal is mainly for the prevention of post-operative inflammation and not for infection prevention. Moreover the study is more focused on the effect of fosfosal phosphates on bioactivity than on its anti-inflammatory action. It is an injectable material.

Biomaterials - Mendez et al. - 2004 vol 25 pp 2381-2392 describes a PMMA-based cement comprising 3 different phases (PMMA, bioactive glass and drug). The anti-inflammatory properties are given by a drug. The addition of the drug fosfosal is mainly for the prevention of post-operative inflammation and not for infection prevention. The cited application for the proposed material is vertebroplasty.

Journal of Materials Science Materials in Medicine - Verne et al. - 2009 vol 20 pp 733-740, draft by the inventors of the present application, regards the use of ion-exchange process to dope with silver a massive bioactive glass. The doped glass in characterized in terms of morphology, composition, structure, silver leaching, bioactivity and antibacterial properties.

Journal of Materials Science Materials in Medicine - Miola et al. - 2009 vol 20 pp 741-749, draft by the inventors of the present application, concerns with the deposition of bio-active glass powders by plasma spray on titanium alloy and stainless steel substrates. The obtained coatings are subjected to ion-exchange treatment to introduce silver ions and characterized by means of morphological-compositional and structural analyses, leaching and bioactivity tests, biocompatibility and antibacterial evaluations.

One object of the present invention is to provide a novel bone cement composition, particularly for use in the fixation of orthopaedic prostheses, in spinal surgery or in the production of temporary prostheses, made of acrylic polymers, which at the same time is suitable to promote binding to the tissue with which it comes into contact, and thus integration of the prosthetic device, and suitable to effect a sustained antibacterial action.

For such a purpose, object of the invention is a bone cement having the characteristics defined in the claims that follow.

In the bone cement object of the invention, the acrylic polymer typically is polymethylmethacrylate (PMMA), but it may also be made of a methylmethacrylate and methylacrylate copolimer, or by a bisphenol-α-glycidylmethacrylate (bis-GMA) polymer or mixtures thereof.

The glass/glass-ceramic component is introduced in the form of a powder into the polymer component of the bone cement. The mixture thus obtained is subsequently polymerised in situ by stirring with the liquid monomer and the activator. Typically, according to the conventional technique, the polymer component and glass/glass-ceramic component mixture preferably contains a powdered X ray opacifier, for example zirconium dioxide and/or barium sulphate.

The liquid fraction contains the monomer, typically methylmethacrylate, in which a radical activator is dissolved such as for example N-N-dimethyl-p-toluidine. It is however understood that the invention is not restricted to the selection of specific initiators and/or radical activators.

The percentage of the glass/glass-ceramic component in the polymerised bone cement generally is lower than 80% by weight, referred to the total weight of the bone cement, and preferably is lower than 50% by weight, so as to allow for an excellent homogenisation with the acrylic polymer component. Percentages of the glass/glass-ceramic component from 10% to 50% by weight are preferred.

The granulometry of the glass powders, referred to as the largest granule size, typically is lower than 80 µm and preferably lower than 20 µm.

The bioactive glass/glass-ceramic component contains at least the following oxides: SiO₂, CaO, Na₂O, as well as at least one silver, zinc and/or copper oxide or mixtures thereof. Preferably, the glass/glass-ceramic component comprises:
- SiO₂: 40-60% by moles;
- CaO: 15-45% by moles;
- Na₂O: 5-25% by moles;
- Ag₂O: 0.1-10% by moles, preferably 0.1-1% by moles; and/or
- ZnO: 0.1-20% by moles, preferably 0.1-10% by moles; and/or
- CuO: 0.1-10% by moles; the total amount of antibacterial metal oxides preferably being not higher than 20% molar.

Further oxides may be added, such as P₂O₅, K₂O, MgO, Al₂O₃ e B₂O₃, individually or as mixtures of two or more of the mentioned oxides. By way of example, each of said oxides may be used in the glass composition in accordance with the following molar concentrations:
- P₂O₅: 0-10% by moles, preferably 1-5% by moles;
- K₂O: 0-10% by moles, preferably 1-10%;
- MgO: 0-10% by moles, preferably 1-8%;
- Al₂O₃: 0-3% by moles, preferably 1-3%;
- B₂O₃: 0-3% by moles, preferably 1-3%;
- CaF₂: 0-10% by moles, preferably 1-9%;

The glass/glass-ceramic component can be obtained by fusion of precursors of the above-mentioned oxides, typically carbonates. Alternatively, the glass/glass-ceramic component can be obtained by the sol-gel process.

The *per se* known sol-gel synthesis method is performed by stirring the metal alkoxides in solution, followed by hydrolysis, gelatinization and baking.

According to the invention, the antibacterial element is inserted into the composition of the glass (or glass-ceramic), after synthesis, through ion exchange processes from solutions, for instance according to the method described in EP 1 819 372.

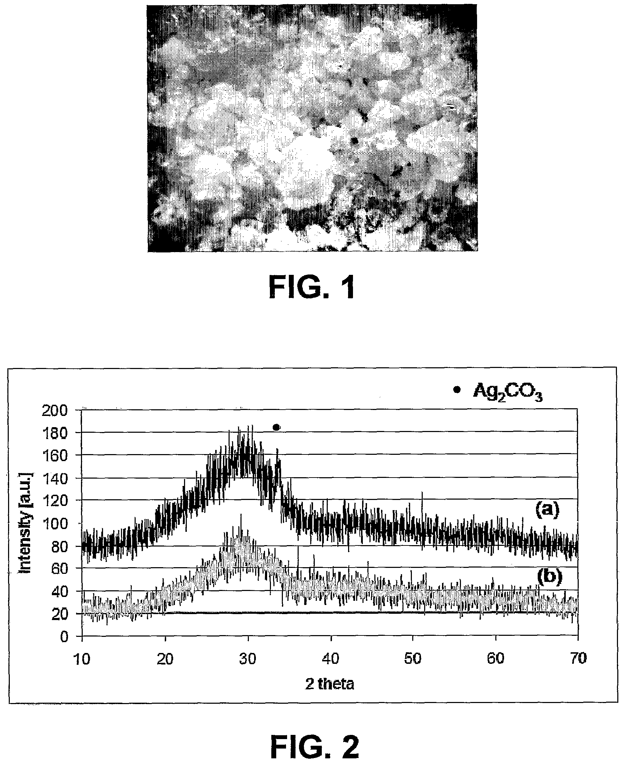

The ion exchange technique allows even high amounts of silver to be introduced into the glasses and glass-ceramics of suitable composition, which is difficult to achieve by the fusion and casting technique. Usually, the bio-glasses are synthesised by using refractory pots, therefore a high silver content may cause interactions between the silver and the pot, with formation of unwanted phases, and consequent non-uniform silver content in the different castings. Furthermore, the fusion and casting technique does not allow for an excellent silver dispersion and homogenisation within the material, with frequent formation of metal clusters (Figure 1).

EP 1 819 372 describes the bulk application of the ion exchange method to glass and/or glass-ceramic materials or on metal coatings. For application on powders, the process parameters need to be suitably modified and controlled; in fact, the same conditions applied for instance to bulks and powders give decidedly different results.

Particularly the ion exchange technique on powders must be carefully examined for each specific glass/glass-ceramic composition employed, even considering parameters, such as for example the pH value of the exchange solutions, which generally do not have effect during the process performed on bulks, coatings and scaffolds.

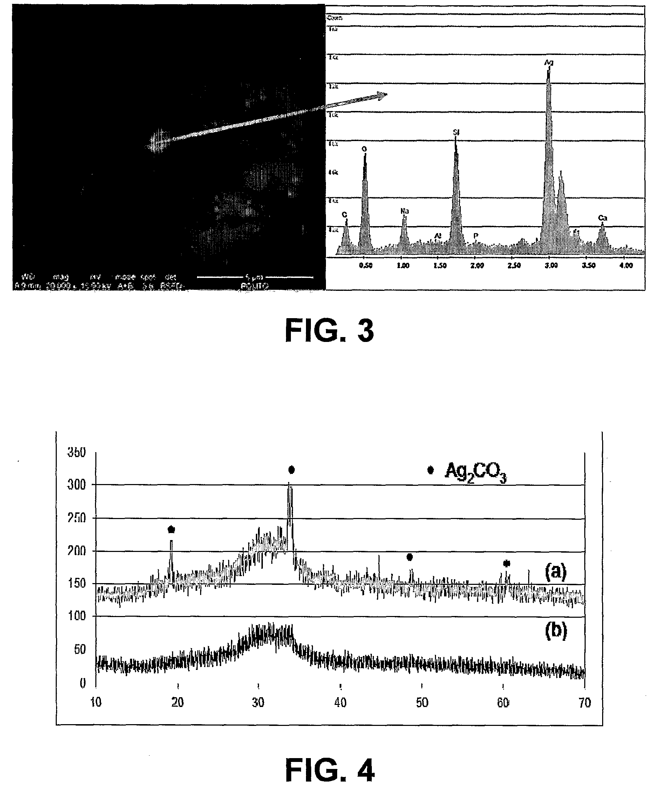

The high specific surface of powders makes them more reactive towards the surrounding environment and particularly during the ion exchange process in solution. For instance, glass powders (Example 1 composition), subjected to ion exchange under the same conditions of bulks and coatings with an identical composition, show precipitation of silver carbonate as a result of reaction between Ag+ ions and CO₂ or the carbonates in the reaction environment (Figure 2.a, Figure 3). The presence of silver carbonate precipitates on the bioactive glass powders, instead of ions diffused therein, does not ensure a gradual and sustained release of silver ions and establishes an uncontrollable phenomenon of a third phase formation. Powders with the same glass composition containing Ag or the other antibacterial ions previously mentioned, without precipitation of second phases, can be obtained only by carefully selecting the process parameters. (Figure 2.b).

As previously anticipated, in order to obtain powders with an adequate dosage of silver ions without precipitation of other phases, not only the normal exchange parameters (time, temperature and concentration of the exchange solution) need to be varied and controlled, but also further parameters such as for example the pH of the exchange solutions.

In fact, precipitation of carbonates is favoured at highly basic pHs; instead, the maintenance of a pH between 5 and 8, preferably comprised between 7 (neutral) and 7.6, allows to favour maintenance of the antibacterial ions (e.g. silver) in solution, and thus a suitable diffusion thereof within the glass particles. Such a pH control could be carried out by adding a buffer to the exchange solutions; this solution however is not applicable to this specific case since it induces formation of other silver salt precipitates (chlorides, phosphates...). In the application according to the invention it is preferable to use a glassy composition, the ion release of which does not make the solution highly basic (Figure 4).

Thus, a relevant feature of the bone cements object of the invention is that the antibacterial agent is only incorporated in the glass or glass-ceramic material and additional phases other than glass or glass-ceramic made of or enclosing the antibacterial agent are absent, such as for example inter-metal phases or metal clusters or precipitates including the anti-bacterial agent.

Therefore, the bone cement composition according to the invention is essentially a monophasic composition with regard to the phases that include the antibacterial agent.

Such a feature allows to obtain a controlled release of the antibacterial ions when the bone cement is applied in situ in contact with physiological fluids.

To that end, the bone cement of the invention is prepared by using glass or glass-ceramic material powder subjected to ion exchange in an aqueous solution containing antibacterial metal ions; the ion exchange process is carried out at a pH comprised between 5 and 8, preferably at temperatures below 100°C, preferably from 37 to 100°C, for periods of from 15 to 240 minutes, , by using a powdered glass or glass-ceramic material that includes metal ions (e.g. alkaline or alkaline earth materials) liable to exchange with the antibacterial ions (particularly silver).

In particular, a bioactive powdered glass or glass-ceramic material is used, the oxide composition of which is such that, when the material is kept in water, the release of chemical species from such a material is not capable of bringing the pH to values higher than 8, under balance conditions or for periods of up to 240 minutes.

Examples of compositions suitable for maintaining a neutral pH:

| | %wt | %mol |
|---|---|---|
| SiO₂ | 49 | 49.81 |
| Na₂O | 24 | 23.61 |
| CaO | 22 | 23.96 |
| P₂O₅ | 3.2 | 1.37 |
| B₂O₃ | 0.6 | 0.53 |
| Al₂O₃ | 1.2 | 0.72 |
| TOT | 100 | 100.00 |
| SiO₂ | 46.53 | 48 |
| Na₂O | 18.03 | 18 |
| CaO | 27.14 | 30 |
| P₂O₅ | 6.88 | 3 |
| B₂O₃ | 0.48 | 0.43 |
| Al₂O₃ | 0.94 | 0.57 |
| TOT | 100.00 | 100 |

Further advantages and features of the bone cement according to the invention will be apparent from the following examples.

In the appended drawings:
- Fig. 1 illustrates a glass frit containing 1%wt of Ag obtained by fusion and casting. The yellow colouring is likely due to the presence of nano-clusters.
- Fig. 2 illustrates the X ray analysis of glass powders with a composition according to Example 1, exchanged under the same conditions as bulks and coatings (a) and under optimised ion exchange conditions (b).
- Fig. 3 reports SEM and EDS analyses of precipitates containing silver on powders exchanged under the same conditions as bulks and coatings.
- Fig. 4 illustrates an XRD analysis of glass powders that bring the pH of the exchange solutions at highly basic values (a) and of glassy powders (prepared according to Example 3) which instead favour the maintenance of a neutral or slightly basic pH (b) exchanged under the same conditions.
- Fig. 5 illustrates SEM micrographs and an EDS analysis of a composite cement prepared according to reference Example 5b;
- Fig. 6 illustrates the inhibition halo of composite cements prepared according to Example 4;
- Fig. 7 illustrates the inhibition halo of composite cements prepared according to reference Example 5b;
- Fig. 8 is a diagram that illustrates the release trend of the silver ion from composite cements prepared according to Example 4.

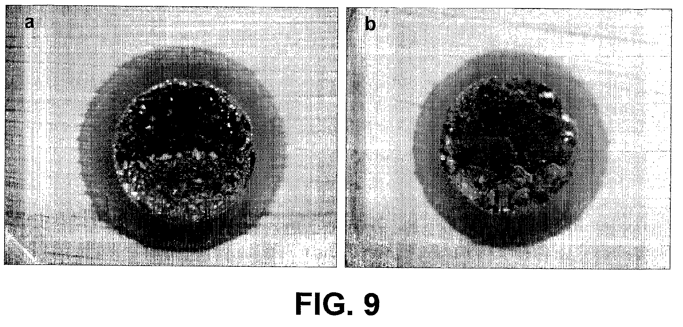

- Fig. 9 illustrates the inhibition halo of low- (a) and high- (b) viscosity composite cements prepared according to Example 6.

### Example 1 - Preparation of the bioactive glass component

A bioactive glass having the following composition was prepared:
- SiO₂: 57% by moles
- CaO: 34% by moles
- Na₂O: 6% by moles
- Al₂O₃: 3% by moles

The glass was prepared by using SiO₂, CaCO₃, Na₂CO₃, Al₂O₃ as the oxide precursors.

The fusion process was performed at a temperature of about 1400°C-1550°C and the molten was poured out into water to obtain powders.

The powder thus obtained was milled and sieved to a size smaller than 20 µm. The powders were added with silver ions by replacement of sodium ions through ion exchange in aqueous silver nitrate solutions, thereby obtaining a final glass composition of:
- SiO₂: 57% by moles
- CaO: 34% by moles
- Na₂O: 5.9% by moles
- Al₂O₃: 3% by moles
- Ag₂O: 0.1% by moles

### Reference Example 2 - Preparation of the bioactive glass component by the fusion process

The synthesis was carried out as in Example 1, by including though directly the silver oxide in the form of Ag₂CO₃ within the precursors, thereby obtaining a glass having the following molar composition:
- SiO₂: 50% by moles
- CaO: 24% by moles
- Na₂O: 22.2 % by moles
- Al₂O₃: 0.7 % by moles
- P₂O₅: 1.4 % by moles
- B₂O₃: 1.4 % by moles
- Ag₂O: 0.3 % by moles

### Example 3 - Preparation of the bioactive glass component.

A bioactive glass having the following composition was prepared:
- SiO₂: 48% by moles
- CaO: 30% by moles
- Na₂O: 18% by moles
- P₂O₅: 3% by moles
- Al₂O₃: 0.57% by moles
- B₂O₃: 0.1% by moles

The glass was prepared as in Example 1 and silver ions were added by replacement of sodium ions through ion exchange in aqueous silver nitrate solutions, as in Example 1.

### Example 4 - Preparation of the cement

The powder obtained as in Example 1 was mixed with a polymeric polymethylmethacrylate component according to the following ratios:
- PMMA: 50% by weight
- glass components: 50% by weight

### Reference Example 5 - Preparation of the cement

The powder obtained as in Example 2 was mixed with a polymeric polymethylmethacrylate component according to the following ratios:

### Example 5a

- PMMA: 70 % by weight
- glass components: 30 % by weight

### Example 5b

- PMMA: 50% by weight
- glass components: 50% by weight

The tests performed demonstrated that the bioactive glasses/glass-ceramics, even when included in the polymeric acrylic composition, are capable of promoting the formation of a hydroxyapatite layer on their surface subsequent to reaction with simulated physiological fluids.

### Example 6 - Preparation of the cement.

The powder obtained as in Example 3 was mixed with a polymeric polymethylmethacrylate component according to the following ratios:
- PMMA: 70 % by weight
- glass components: 30 % by weight

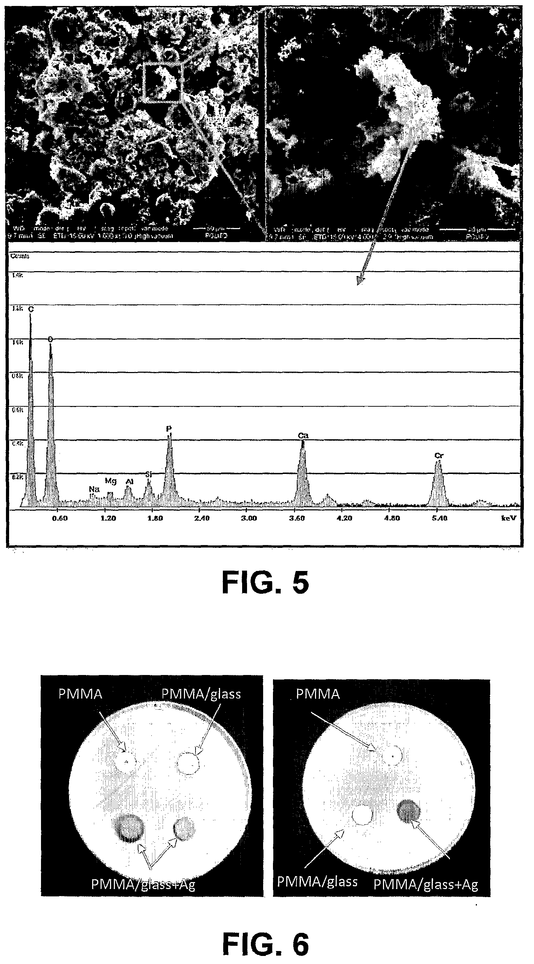

A micrograph of the surface of a composite cement prepared according to reference Example 5b is reported by way of example, where formation of hydroxyapatite is observed after immersion in a simulated physiological solution for 28 days (Fig. 5).

The presence of a bioactive phase that becomes exposed on the cement surface causes this structure to promote the binding in vivo to the tissue with which it comes into contact, and thus integration of the prosthesis thus cemented. The presence of the glass/glass-ceramic component also has the advantage of decreasing the local temperature rise due to the exothermic character of the polymerisation reaction, with undisputed advantages for the bone directly in contact with the cement.

Moreover, adding the second glass/glass-ceramic phase does not alter the material's processing and hardening properties

In addition, the presence of the inorganic phase in the bone cement contributes to enhancing the cement's mechanical properties.

By suitably varying the glass composition and the parameters for the introduction of the antibacterial element, it is possible to modulate the bioactivity degree of the cement, the release kinetics of the metal ions and the mechanical properties of the composite material, depending on the particular application requirements.

The antibacterial tests on the bone cement were carried out by using each of the cement types mentioned in Examples 4, 5, 6.

The antibacterial effect of the composite cements was assessed by the inhibition halo test according to the NCCLS (National Committee for Clinical Laboratory) standards. Such a trial contemplates preparing a solution of known bacterial concentration and diffusing an aliquot of such a solution onto Mueller Hinton plates, which allow the bacteria to grow rapidly. The samples are placed on the plates containing the bacteria and incubated at 35°C for 24 hours. At the end of the incubation the area where the bacteria did not grow is examined and measured.

The antibacterial tests were carried out by using a standard *Staphylococcus aureus* strain, one of the bacterial strains most involved in the development of infections.

Fig. 6 shows a comparison between a bone cement manufactured according to Example 4 and a cement loaded with powders from the same bioactive glass without silver oxide.

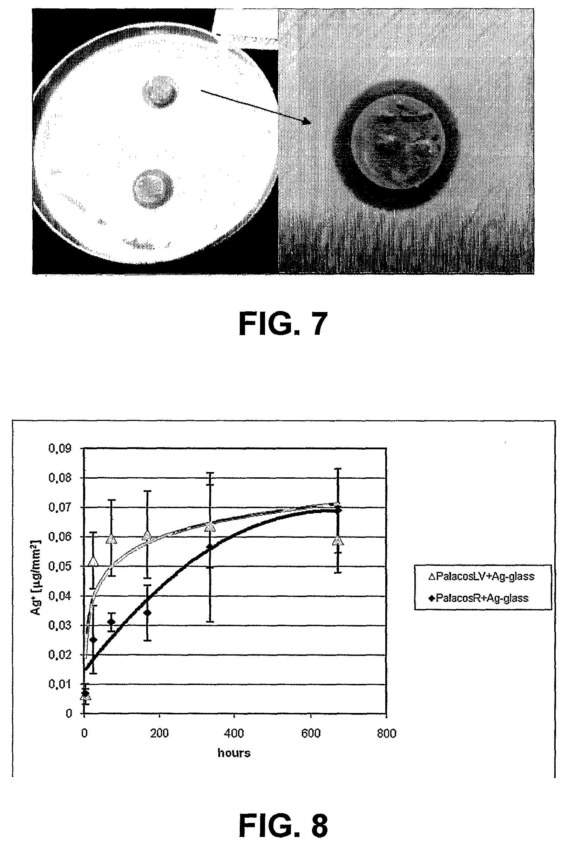

Fig. 7 illustrates the antibacterial effect obtained with the cements described in reference Example 5b. Fig. 8 shows the antimicrobial capacity of cements described in Example 6 containing the glass dosed with silver through ion exchange with optimised parameters (Example 3).

The test results further demonstrate that the bone cement according to the invention allows to obtain a sustained release of antibacterial metal ions with an activity that lasts for periods of from about 7 days to more than a month and therefore results advantageous compared to the restricted antibacterial activity periods of bone cements loaded with antibiotics.

## Claims

1. A bone cement comprising an acrylic polymeric component and an inorganic component comprising a bioactive glass or glass-ceramic powder material which comprises at least one oxide of a metal having an anti-bacterial activity, wherein said glass or glass-ceramic material is adapted to release ions of said antibacterial metal in contact with physiological fluids, **characterised in that** said antibacterial metal is incorporated in said glass or glass-ceramic powder material by subjecting said powder to ion exchange in an aqueous solution, at a pH between 5 and 8, containing ions of said anti-bacterial metal and wherein, in the bone cement, additional phases including said antibacterial metal, other than said glass or glass-ceramic powder material, are absent.

2. A bone cement according to claim 1, **characterised in that** it comprises:
- from 10% to 80% by wt. of the glass or glass-ceramic component and
- from 20% to 90% by wt. of acrylic polymeric component, referred to the total weight of said components.

3. A bone cement according to claims 1 or 2, **characterised in that** the glass or glass-ceramic component comprises:
- SiO₂: from 40% to 60% by moles;
- CaO: from 15% to 45% by moles;
- Na₂O: from 5% to 25% by moles;
and at least one oxide selected from silver oxides, zinc oxide and copper oxide and mixtures thereof.

4. A bone cement according to claim 3, **characterised in that** it comprises one or more of the following oxides:
- Ag₂O: from 0.1 % to 10% by moles, preferably from 0.1 % to 1% by moles;
- ZnO: from 0.1% to 20% by moles, preferably from 0.1% to 10% moles;
- CuO: from 0.1 % to 10% by moles, preferably from 0.1 % to 1% by moles;
or mixtures of said oxides, the amount of anti-bacterial metal oxides being not higher than 20% by moles.

5. A bone cement according to claims 3 or 4, **characterised in that** it further comprises one or more of the following compounds or mixtures thereof:
- P₂O₅: from 0% to 10% by moles, preferably from 1% to 5%;
- K₂O: 0-10% by moles, preferably from 1% to 10%;
- MgO: 0-10% by moles, preferably from 1% to 8%;
- Al₂O₃: 0-3% by moles, preferably from 1% to 3%;
- B₂O₃: 0-3% by moles, preferably from 1% to 3%;
- CaF₂: 0-10% by moles, preferably from 1% to 9%.

6. A bone cement according to any of the preceding claims, **characterised in that** the polymeric component comprises polymethylmethacrylate and/or methylmethacrylate and methylacrylate copolymers or a polymer of bisphenol-alpha-glycidylmethacrylate.

7. A composition for the production of a bone cement comprising:
- a powder phase including an acrylic polymer component and a glass or glass-ceramic powder material component, which encloses an oxide of an antibacterial metal, optionally including a polymerisation initiator and/or a radio-opacifying agent; and,
- a liquid phase including an acrylic monomer and optionally a polymerisation accelerator and/or inhibitor, and wherein the antibacterial metal is only enclosed within the glass or glass-ceramic component, any additional phase including said antibacterial metal being absent, and wherein said antibacterial metal is incorporated in said glass or glass-ceramic powder material by subjecting said powder to ion exchange in an aqueous solution at a pH between 5 and 8 containing ions of said anti-bacterial metal.

8. A composition according to claim 7, **characterised in that** said powder phase comprises:
- from 10% to 80% by wt. of glass or glass-ceramic component; and
- from 20% to 90% by wt. of acrylic polymeric component, referred to the total weight of said components.

9. A composition according to claim 8, **characterised in that** said glass or glass-ceramic component comprises from 10% to 50% by wt. referred to the total weight of said powder phase.

10. A bone cement or a composition for the manufacture of a bone cement, according to any of claims 1 to 6, wherein said glass or glass-ceramic component exhibits an oxide composition such that when kept in water, the release of chemical species from such a component is not capable of bringing the pH to values higher than 8, under balance conditions or for periods of less than 240 minutes.

11. A method for the manufacture of a bone cement according to any of claims 1 to 6, comprising mixing at least one acrylic polymer component and one powdered bioactive glass or glass-ceramic powder material component enclosing an antibacterial metal, **characterised in that** said glass or glass-ceramic component is obtained by an ion exchange process in an aqueous solution including one or more ions of an antibacterial material, applied to a powder of said component, under pH conditions kept between 5 and 8.

12. The method according to claim 11, wherein said glass or glass-ceramic component exhibits a metal oxide composition such that when kept in water, the release of chemical species from said component is not capable of bringing the pH to values higher than 8, under balance conditions for periods of less than 240 minutes.

13. A bone cement according to any of claims 1 to 6 or a composition for the manufacture of a bone cement according to any of claims 7 to 9, for use in a surgical treatment for the fixation of orthopaedic prostheses.

14. A bone cement according to any of claims 1 to 6, for use in a spinal surgery treatment as a reinforcement cementing material.

15. A temporary prosthesis produced with the use of a bone cement according to any of claims 1 to 6.

## Patentansprüche

1. Knochenzement, der eine Acrylpolymerkomponente und eine anorganische Komponente umfasst, die ein bioaktives Glas- oder Glaskeramikpulvermaterial umfasst, das mindestens ein Oxid eines Metalls umfasst, das eine antibakterielle Aktivität aufweist, wobei das Glas- oder Glaskeramikmaterial zum Freisetzen von Ionen des antibakteriellen Metalls in Kontakt mit physiologischen Fluiden angepasst ist, **dadurch gekennzeichnet, dass** das antibakterielle Metall dadurch in das Glas- oder Glaskeramikpulvermaterial einbezogen wurde, dass das Pulver einem Ionenaustausch in einer wässrigen Lösung bei einem pH-Wert zwischen 5 und 8, die Ionen des antibakteriellen Metalls enthält, unterzogen worden ist, und wobei in dem Knochenzement zusätzliche Phasen, die das antibakterielle Metall enthalten, die von dem Glas- oder Glaskeramikpulvermaterial verschieden sind, nicht vorliegen.

2. Knochenzement nach Anspruch 1, **dadurch gekennzeichnet, dass** er umfasst:
- von 10 Gew.-% bis 80 Gew.-% der Glas- oder Glaskeramikkomponente und
- von 20 Gew.-% bis 90 Gew.-% der Acrylpolymerkomponente, bezogen auf das Gesamtgewicht der Komponenten.

3. Knochenzement nach Anspruch 1 oder 2, **dadurch gekennzeichnet, dass** die Glas- oder Glaskeramikkomponente umfasst:
- SiO₂: von 40 mol-% bis 60 mol-%,
- CaO: von 15 mol-% bis 45 mol-%,
- Na₂O: von 5 mol-% bis 25 mol-%,
und mindestens ein Oxid, das aus Silberoxiden, Zinkoxid und Kupferoxid und Gemischen davon ausgewählt ist.

4. Knochenzement nach Anspruch 3, **dadurch gekennzeichnet, dass** er eines oder mehrere der folgenden Oxide umfasst:
- Ag₂O: von 0,1 mol-% bis 10 mol-%, vorzugsweise von 0,1 mol-% bis 1 mol-%,
- ZnO: von 0,1 mol-% bis 20 mol-%, vorzugsweise von 0,1 mol-% bis 10 mol-%,
- CuO: von 0,1 mol-% bis 10 mol-%, vorzugsweise von 0,1 mol-% bis 1 mol-%,
oder Gemische der Oxide, wobei die Menge der antibakteriellen Metalloxide nicht höher als 20 mol-% ist.

5. Knochenzement nach Anspruch 3 oder 4, **dadurch gekennzeichnet, dass** er ferner eine oder mehrere der folgenden Verbindungen oder Gemische davon umfasst:
- P₂O₅: von 0 mol-% bis 10 mol-%, vorzugsweise von 1 % bis 5 %,
- K₂O: von 0 mol-% bis 10 mol-%, vorzugsweise von 1 % bis 10 %,
- MgO: von 0 mol-% bis 10 mol-%, vorzugsweise von 1 % bis 8 %,
- Al₂O₃: von 0 mol-% bis 3 mol-%, vorzugsweise von 1 % bis 3 %,
- B₂O₃: von 0 mol-% bis 3 mol-%, vorzugsweise von 1 % bis 3 %,
- CaF₂: von 0 mol-% bis 10 mol-%, vorzugsweise von 1 % bis 9 %.

6. Knochenzement nach einem der vorhergehenden Ansprüche, **dadurch gekennzeichnet, dass** die polymere Komponente Polymethylmethacrylat und/oder Methylmethacrylat- und Methylacrylat-Copolymere oder ein Polymer von Bisphenol-alpha-Glycidylmethacrylat umfasst.

7. Zusammensetzung zur Herstellung eines Knochenzements, umfassend:
- eine Pulverphase, die eine Acrylpolymerkomponente und eine Glas- oder Glaskeramikpulvermaterialkomponente umfasst, die ein Oxid eines antibakteriellen Metalls einschließt, und die gegebenenfalls einen Polymerisationsinitiator und/oder ein strahlenundurchlässig machendes Mittel umfasst, und
- eine flüssige Phase, die ein Acrylmonomer und gegebenenfalls einen Polymerisationsbeschleuniger und/oder -hemmstoff umfasst, wobei das antibakterielle Metall nur innerhalb der Glas- oder Glaskeramikkomponente eingeschlossen ist, jedwede zusätzliche Phase, die das antibakterielle Metall umfasst, fehlt, und wobei das antibakterielle Metall dadurch in das Glas- oder Glaskeramikpulvermaterial einbezogen wurde, dass das Pulver einem Ionenaustausch in einer wässrigen Lösung bei einem pH-Wert zwischen 5 und 8, die Ionen des antibakteriellen Metalls enthält, unterzogen worden ist

8. Zusammensetzung nach Anspruch 7, **dadurch gekennzeichnet, dass** die Pulverphase umfasst:
- von 10 Gew.-% bis 80 Gew.-% einer Glas- oder Glaskeramikkomponente und
- von 20 Gew.-% bis 90 Gew.-% einer Acrylpolymerkomponente, bezogen auf das Gesamtgewicht der Komponenten.

9. Zusammensetzung nach Anspruch 8, **dadurch gekennzeichnet, dass** die Glas- oder Glaskeramikkomponente von 10 Gew.-% bis 50 Gew.-% bezogen auf das Gesamtgewicht der Pulverphase ausmacht.

10. Knochenzement oder Zusammensetzung zur Herstellung eines Knochenzements nach einem der Ansprüche 1 bis 6, wobei die Glas- oder Glaskeramikkomponente eine Oxidzusammensetzung aufweist, die derart ist, dass dann, wenn sie in Wasser gehalten wird, die Freisetzung von chemischen Spezies von einer solchen Komponente nicht in der Lage ist, unter Gleichgewichtsbedingungen oder für Zeiträume von weniger als 240 Minuten den pH-Wert auf Werte von höher als 8 zu bringen.

11. Verfahren zur Herstellung eines Knochenzements nach einem der Ansprüche 1 bis 6, welches das Mischen von mindestens einer Acrylpolymerkomponente und einer pulverförmigen bioaktiven Glas- oder Glaskeramikpulvermaterialkomponente, die ein antibakterielles Metall einschließt, umfasst, **dadurch gekennzeichnet, dass** die Glas- oder Glaskeramikkomponente durch ein lonenaustauschverfahren in einer wässrigen Lösung, die ein oder mehrere Ion(en) eines antibakteriellen Materials umfasst, das auf ein Pulver der Komponente angewandt wird, bei pH-Bedingungen erhalten wird, die zwischen 5 und 8 gehalten werden.

12. Verfahren nach Anspruch 11, bei dem die Glas- oder Glaskeramikkomponente eine Metalloxidzusammensetzung aufweist, die derart ist, dass dann, wenn sie in Wasser gehalten wird, die Freisetzung von chemischen Spezies von der Komponente nicht in der Lage ist, unter Gleichgewichtsbedingungen für Zeiträume von weniger als 240 Minuten den pH-Wert auf Werte von höher als 8 zu bringen.

13. Knochenzement nach einem der Ansprüche 1 bis 6 oder Zusammensetzung zur Herstellung eines Knochenzements nach einem der Ansprüche 7 bis 9 zur Verwendung in einer chirurgischen Behandlung zur Fixierung von orthopädischen Prothesen.

14. Knochenzement nach einem der Ansprüche 1 bis 6 zur Verwendung als Verstärkungszementierungsmaterial in einer Spinalchirurgiebehandlung.

15. Temporäre Prothese, die unter Verwendung eines Knochenzements nach einem der Ansprüche 1 bis 6 hergestellt worden ist.

## Revendications

1. Ciment osseux constitué d'un composant acrylique polymérique et d'un composant inorganique constitué d'une poudre de verre ou vitrocéramique bio-actif, comprenant au moins un oxyde de métal doté d'une activité antibactérienne, dans lequel ledit matériau de verre ou vitrocéramique est adapté pour libérer des ions dudit métal antibactérien au contact des liquides physiologiques, **caractérisé en ce que** ledit métal antibactérien est incorporé dans ledit matériau de verre ou vitrocéramique en soumettant ladite poudre à un échange d'ions dans une solution aqueuse, au pH compris entre 5 et 8, contenant des ions dudit métal antibactérien et dans lequel, dans le ciment osseux, d'autres phases, y compris ledit métal antibactérien, autres que ladite poudre de verre ou vitrocéramique, sont absentes.

2. Ciment osseux selon la revendication 1, **caractérisé en ce qu'**il comprend :
- de 10% à 80% en poids de composant de verre ou vitrocéramique et
- de 20% à 90% en poids de composant acrylique polymérique, par rapport au poids total desdits composants.

3. Ciment osseux selon la revendication 1 ou 2, **caractérisé en ce que** le composant de verre ou vitrocéramique comprend :
- du SiO₂ : de 40% à 60% en moles
- du CaO : de 15% à 45% en moles
- du Na₂O : de 5% à 25% en moles
et au moins un oxyde sélectionné parmi les oxydes d'argent, l'oxyde de zinc et l'oxyde de cuivre, et leurs mélanges.

4. Ciment osseux selon la revendication 3, **caractérisé en ce qu'**il comprend un ou plusieurs des oxydes suivants :
- Ag₂O : de 0,1% à 10% en moles, de préférence de 0,1% à 1% en moles
- ZnO : de 0,1% à 20% en moles, de préférence de 0,1% à 10% en moles
- CuO : de 0,1% à 10% en moles, de préférence de 0,1% à 1% en moles ou des mélanges desdits oxydes, la quantité d'oxydes de métal antibactérien n'étant pas supérieure à 20% en moles.

5. Ciment osseux selon les revendications 3 ou 4, **caractérisé en ce qu'**il comprend en outre un ou plusieurs des composés ou mélanges de composés suivants :
P₂O₅ : de 0% à 10% en moles, de préférence de 1% à 5% en moles K₂O : 0% - 10% en moles, de préférence de 1% à 10%
MgO : 0% - 10% en moles, de préférence de 1% à 8%
Al₂O₃ : 0% - 3% en moles, de préférence de 1% à 3%
B₂O₃ : 0% - 3% en moles, de préférence de 1% à 3%
CaF₂ : 0% - 10% en moles, de préférence de 1% à 9%

6. Ciment osseux selon l'une quelconque des revendications précédentes, **caractérisé en ce que** le composant polymérique comprend des copolymères de polyméthylméthacrylate et/ou de méthylméthacrylate et de méthylacrylate ou un polymère de bisphénol-alphaglycidylméthacrylate.

7. Composition pour la production d'un ciment osseux, comprenant :
- une phase poudre incluant un composant acrylique polymérique et un composant poudre de verre ou vitrocéramique, qui renferme un oxyde d'un métal antibactérien, incluant facultativement un initiateur de polymérisation et /ou un agent radio-opacifiant ; et
- une phase liquide incluant un monomère acrylique et, facultativement, un accélérateur et/ou un inhibiteur de polymérisation, et dans laquelle le métal antibactérien est englobé uniquement dans le composant de verre ou vitrocéramique, toute phase additionnelle incluant ledit métal antibactérien étant absente, et dans laquelle ledit métal antibactérien est incorporé dans ladite poudre de verre ou vitrocéramique en soumettant ladite poudre à un échange d'ions dans une solution aqueuse de pH compris entre 5 et 8, contenant des ions dudit métal antibactérien.

8. Composition selon la revendication 7, **caractérisée en ce que** ladite phase poudre comprend :
- de 10% à 80% en poids de composant de verre ou vitrocéramique, et
- de 20% à 90% en poids de composant acrylique polymérique, par rapport au poids total desdits composants.

9. Composition selon la revendication 8, **caractérisée en ce que** ledit composant de verre ou vitrocéramique comprend de 10% à 50% en poids, par rapport au poids total de ladite phase poudre.

10. Ciment osseux ou composition pour la fabrication d'un ciment osseux selon l'une quelconque des revendications 1 à 6, dans lequel ledit composant de verre ou vitrocéramique présente une composition en oxydes telle que, lorsqu'il est mis dans l'eau, la libération d'espèces chimiques par un tel composant n'est pas capable d'amener le pH à des valeurs supérieures à 8, dans des conditions d'équilibre ou pendant des périodes de moins de 240 minutes.

11. Procédé pour la fabrication d'un ciment osseux selon l'une quelconque des revendications 1 à 6, consistant à mélanger au moins un composant acrylique polymérique et un composant poudre de verre ou vitrocéramique bio-actif renfermant un métal antibactérien, **caractérisé en ce que** ledit composant de verre ou vitrocéramique est obtenu par un processus d'échange d'ions dans une solution aqueuse incluant un ou plusieurs ions d'un matériau antibactérien, appliqué à une poudre dudit composant, dans des conditions de pH maintenues entre 5 et 8.

12. Procédé selon la revendication 11, dans lequel ledit composant de verre ou vitrocéramique présente une composition en oxydes métalliques telle que, lorsqu'il est mis dans l'eau, la libération d'espèces chimiques par un tel composant n'est pas capable d'amener le pH à des valeurs supérieures à 8, dans des conditions d'équilibre ou pendant des périodes de moins de 240 minutes.

13. Ciment osseux selon l'une quelconque des revendications 1 à 6, ou composition pour la fabrication d'un ciment osseux selon l'une quelconque des revendications 7 à 9, pour usage dans un traitement chirurgical pour la fixation de prothèses orthopédiques.

14. Ciment osseux selon l'une quelconque des revendications 1 à 6, pour usage en chirurgie rachidienne comme matériau cimentant de renfort.

15. Prothèse temporaire produite en utilisant un ciment osseux selon l'une quelconque des revendications 1 à 6.
